Europäisches Patentamt

(19) European Patent Office

Office européen des brevets

(11) Veröffentlichungsnummer: **0 026 417**
**B1**

(12) **EUROPÄISCHE PATENTSCHRIFT**

(45) Veröffentlichungstag der Patentschrift:
**14.12.83**

(21) Anmeldenummer: **80105661.5**

(22) Anmeldetag: **20.09.80**

(51) Int. Cl.³: **A 61 M 1/03**, B 04 B 5/02,
B 01 D 21/26 // B04B5/02

(54) Zentrifuge mit Blutbeutelsystem zur Trennung von Blutkomponenten.

(30) Priorität: **22.09.79 DE 2938367**
**15.11.79 DE 2946198**
**01.04.80 DE 3012708**

(43) Veröffentlichungstag der Anmeldung:
**08.04.81 Patentblatt 81/14**

(45) Bekanntmachung des Hinweises auf die Patenterteilung:
**14.12.83 Patentblatt 83/50**

(84) Benannte Vertragsstaaten:
**AT CH DE FR GB IT LI NL SE**

(56) Entgegenhaltungen:
**EP - A - 0 019 038**
**DE - A - 2 404 036**
**DE - A - 3 012 227**
**GB - A - 1 569 219**
**US - A - 3 868 321**

(73) Patentinhaber: **Firma Andreas Hettich,**
**Gartenstrasse 100, D-7200 Tuttlingen (DE)**

(72) Erfinder: **Eberle, Günter, Gartenstrasse 100,**
**D-7200 Tuttlingen (DE)**

(74) Vertreter: **Riebling, Günter, Dr. et al, Patentanwälte**
**Dr.-Ing., Dipl.-Ing., Ing.(grad) G. Riebling Dr.-Ing.,**
**Dipl.-Ing. P. Riebling Rennerle 10 Postfach 3160,**
**D-8990 Lindau (Bodensee) (DE)**

ACTORUM AG

## Zentrifuge mit Blutbeutelsystem zur Trennung von Blutkomponenten

Die Erfindung bezieht sich auf eine Zentrifuge mit wenigstens zwei Kammern für miteinander über einen Schlauch verbundene Blutbeutel, wobei in einer der Kammern zusätzlich ein mit Druck beaufschlagbarer Verdrängungskörper eingesetzt ist, der bei Beaufschlagung mit Druck das Volumen des ihm zugeordneten Blutbeutels verringert und dabei dessen Inhalt über den Schlauch in den oder die weiteren Blutbeutel drückt.

Bei einem derartigen Blutbeutelsystem wird die Trennung des Vollblutes in verschiedenen Blutkomponenten während des Zentrifugiervorganges vorgenommen. Über der sich am Boden des einen Blutbeutels absetzenden Schicht von Erythrozyten (nachfolgend nur noch mit Erys bezeichnet) bildet sich eine Übergangsschicht (Buffycoat) auf dem wiederum das Plasma aufsitzt. Es ist eine Zentrifuge mit Blutbeutelsystem zur Trennung von Blutkomponenten gemäss dem Oberbegriff des Patentanspruchs 1 bekannt, (DE-A-2 404 036) bei der der das Vollblut enthaltende Blutbeutel vollständig von einer aufblasbaren Membrane umhüllt ist. Mittels einer Pumpe kann über eine Leitung der Raum ausserhalb der Membrane und innerhalb des Gehäuses unter Druck gesetzt werden. Dabei wird das Volumen des Blutbeutels verringert und dessen Inhalt strömt über eine Leitung in einen anderen Blutbeutel.

Hierbei können aber Fehler insofern auftreten, als Abdichtungsprobleme zwischen der Membrane und dem Durchbruch für die Leitung auftreten können. Über dies ist ein solches System nur für Blutbeutel einer bestimmten Grösse verwendbar.

Die Erfindung hat sich die Aufgabe gestellt eine Zentrifuge mit einem Verdrängungskörper nach den Merkmalen des Oberbegriffs so weiter zu bilden, dass bei seinem Einsatz praktisch keine Abdichtungsprobleme auftreten und die beabsichtigte Trennung des zentrifugierten Inhalts des betreffenden Blutbeutels in einwandfreier Weise vonstatten geht.

Zur Lösung der gestellten Aufgabe ist die Erfindung dadurch gekennzeichnet, dass der Verdrängungskörper als Druckkissen ausgebildet ist, das in der Kammer neben dem Blutbeutel angeordnet ist.

Der Verdrängungskörper ist also als gesondertes Bauelement ausgebildet, das mitsamt seiner Anschlussleitung in die Aufnahme neben dem betreffenden Blutbeutel eingesetzt werden muss; somit entfallen sämtliche Abdichtungsprobleme zum Schlauchansatz, wie sie vorher bei der DE-A-2 404 036 gegeben waren.

Erfindungsgemäss erfolgt die Verdrängung der abzentrifugierten Blutkomponenten aus dem ersten Blutbeutel dadurch, dass der erste Blutbeutel zusammen mit einem mit Druck beaufschlagbaren Druckkissen in einer ersten Kammer des Rotors eingelegt wird. Das Druckkissen ist verformbar und kann entsprechend dem wirkenden Druck sein Volumen vergrössern. Weil das Druckkissen zusammen mit dem ersten Blutbeutel in einer Kammer des Rotors angeordnet ist, wird bei Volumenvergrösserung des Druckkissens der erste Blutbeutel gleichermassen in seinem Volumen verringert. Wenn nach einigen Minuten Laufzeit der Zentrifuge die abzentrifugierten Blutkomponenten im ersten Blutbeutel sich niedergeschlagen haben, wird das Druckkissen in seinem Volumen vergrössert, wodurch der erste Blutbeutel komprimiert wird und zumindest die dem Anschlussschlauch nächstliegende Blutkomponente über den Anschlussschlauch in den in der benachbarten Kammer angeordneten zweiten Blutbeutel hineinverdrängt wird.

In einer ersten Ausführungsform der vorliegenden Erfindung ist es vorgesehen, dass in die Kammer des Rotors einfach der erste mit Blutflüssigkeit gefüllte Blutbeutel zusammen mit einem Druckkissen eingelegt werden.

In einer zweiten Ausführungsform der vorliegenden Erfindung ist es vorgesehen, dass der erste mit Blutflüssigkeit gefüllte Blutbeutel in einen elastisch verformbaren Einsatzbecher eingesetzt ist und der Einsatzbecher in die Kammer des Zentrifugenbechers eingesetzt ist und dort einen Zwischenraum für den Einsatz des Druckkissens freilässt. Der erste, mit Blutflüssigkeit gefüllte Blutbeutel befindet sich also nicht — wie beim erstgenannten Ausführungsbeispiel – in unmittelbarem Körperkontakt mit dem Druckkissen. Vielmehr ist hier ein Einsatzbecher dazwischen geschaltet. Die Verwendung eines Einsatzbechers hat mehrere Vorteile. Zunächst ist der mit Blutflüssigkeit gefüllte erste Blutbeutel in seiner aufrechten Lage im Zentrifugenbecher fixiert, so dass auch im wesentlichen das nicht mit Blut gefüllte Kopfstück des Blutbeutels gerade und aufrecht auch während des Zentrifugiervorganges gehalten wird. Dies ist zur Gewinnung reiner Blutkomponenten wichtig, weil sich im anderen Falle, wenn das Kopfstück umklappt, im Kopfstück selbst Blutflüssigkeit ansammeln kann, die am Zentrifugiervorgang nicht teilnimmt, wobei dann bei der nachfolgenden Verdrängung und bei der Trennung der abzentrifugierten Blutkomponenten diese Blutflüssigkeit das zuerst herausgedrückte Blutplasma verunreinigt. Hierbei wird es also vorgesehen, wenn die Höhe des Einsatzbechers etwa der Länge des Blutbeutels entspricht.

In einer weiteren Ausführungsform der vorliegenden Erfindung ist es vorgesehen, dass bereits schon vor Beginn des Zentrifugiervorganges das Druckkissen mit Druck beaufschlagt wird, um dementsprechend den mit Blut gefüllten Blutbeutel zu komprimieren. Hierdurch wird der Blutbeutel starr und aufrecht im Einsatzbecher innerhalb des Zentrifugenbechers der Zentrifuge fixiert, so dass ein Umklappen des Kopfstückes mit Sicherheit vermieden wird.

In den oben beschriebenen Ausführungsbeispielen war es vorgesehen, dass die Blutbeutel (mit Blutflüssigkeit gefüllte Blutbeutel und Satelliten-Blutbeutel) aufrecht stehend oder hängend im Zentrifugenbecher des Rotors der Zentrifuge angeordnet waren.

In einer anderen Ausführungsform der vorliegenden Erfindung ist es vorgesehen, dass die Blutbeutel horizontal liegend in getrennten Kammern des Rotors der Zentrifuge angeordnet sind. Hierbei gibt es verschiedene Möglichkeiten:

In einer ersten Anordnung sind die ersten und zweiten Blutbeutel in einer einzigen horizontalen Ebene nebeneinander angeordnet, wobei zwischen zwei mit Blutflüssigkeit gefüllten Blutbeuteln jeweils ein Druckkissen angeordnet ist.

In einer anderen Ausführungsform sind die mit Blutflüssigkeit gefüllten Blutbeutel in einer ersten horizontalen Ebene im Rotor des Zentrifugenbechers angeordnet, während die (Satelliten-) Blutbeutel in einer darunter- oder darüberliegenden zweiten horizontalen Ebene angeordnet sind.

Während des Zentrifugiervorganges muss auf jeden Fall ein Abknicken des Anschlussteils des Blutbeutels vermieden werden, weil sich auch im Anschlussteil des Blutbeutels, d.h. in dem Teil, in dem die Anschlussschläuche und sonstige Anschlussöffnungen münden, Blutflüssigkeit befindet. Knickt dieser Anschlusteil während des Zentrifugiervorganges ab, was leicht geschehen kann, weil der Blutbeutel während des Zentrifugiervorganges mit in axialer Richtung gerichteter Zentrifugalkraft gegen den Boden des Zentrifugenbechers gepresst wird, dann wird in diesem Teil Blutflüssigkeit eingeschlossen, die dann nicht mehr am Zentrifugiervorgang teilnimmt.

Wenn während oder nach dem Zentrifugiervorgang das Plasma dann über den Anschlusteil und die Anschlussschläuche in den Satelliten-Blutbeutel geleitet werden soll, so nimmt dieses Plasma die vorher in dem umgeknickten Anschlusteil befindliche Blutflüssigkeit mit, wodurch es verunreinigt wird. Die Qualität und Reinheit des gewonnenen Blutplasmas ist dadurch stark beeinträchtigt.

Zur Vermeidung solcher sogenannter Sedimentationsfallen ist es bekannt, in entsprechende Ausnehmungen des Blutbeutels federbelastete Haken einzusetzen, die den Blutbeutel während des Zentrifugiervorganges in aufrechter Lage federbelastet halten, und os ein Umknicken vermeiden. Nachteil dieser Anordnung ist jedoch, dass das Aufsetzen und Befestigen des Blutbeutels an diesen Haken relativ umständlich ist, und dass die zugeordneten Ausnehmungen des Blutbeutels zum Ausreissen neigen. Ferner ist es mit den bekannten – zum Stand der Technik gehörenden – Lösungen nicht möglich, eine Kompression des Blutbeutels während des Zentrifugiervorganges derart vorzunehmen, dass nach Vorliegen der durch Abzentrifugieren gewonnenen Blutbestandteile im Blutbeutel während des Zentrifugiervorganges eine Überleitung von Blutplasma in den benachbart angeordneten Satelliten-Blutbeutel möglich ist.

Die vorgeschlagene Lösung hat daher den Vorteil, dass mit Sicherheit ein Umknicken oder Umlegen des Anschlussteils des Blutbeutels während des Zentrifugiervorganges vermieden wird.

Gleichzeitig ist während des Zentrifugiervorganges eine Kompression des Blutbeutels möglich und damit besteht die Möglichkeit, während oder nach dem Abzentrifugieren die gewonnenen Blutbestandteile bei rotierender Zentrifuge in einen benachbarten Satelliten-Blutbeutel zu befördern. Dies erfolgt mit einem Halterungsteil, das lösbar mit dem Anschlussteil des Blutbeutels verbindbar ist, dessen Aussenumfang dem Innenumfang des Zentrifugenbechers angepasst ist, und das in axialer Richtung (Pfeilrichtung) im Zentrifugenbecher bewegbar ist.

Wesentliches Merkmal der vorliegenden Erfindung ist also, dass der Blutbeutel mit der vorgeschlagenen Vorrichtung sicher und beschädigungsfrei in dem Zentrifugenbecher aufrecht gehalten wird. Es erfolgt hier eine vergleichsweise schwimmende Halterung des Blutbeutels, ohne dass es zu einem Ausreissen oder einer sonstigen Beschädigung des Blutbeutels kommt. Man muss jetzt auch nicht mehr für jeden Blutbeutel getrennte Halterungen verwenden, so wie es vorher bei den beschriebenen, federbelasteten Haken der Fall war. Die vorgeschlagene Vorrichtung ist also universell einsetzbar und hängt nicht von der Grösse und Beschaffenheit der Blutbeutel ab.

Mit der vorgeschlagenen technischen Lehre ergibt sich gleichzeitig der Vorteil, dass mit Hilfe des vorgeschlagenen Halterungsteils der Blutbeutel während des Zentrifugiervorganges aufrecht gehalten wird, und daher das Anschlussteil vor dem Abknicken bewahrt wird; darüber hinaus ist das Halterungsteil aber auch noch in axialer Richtung im Zentrifugenbecher bewegbar.

Das Halterungsteil übt damit auf den Blutbeutel eine Kolben-ähnliche Wirkung aus, wobei der zugeordnete Zylinder durch den Zentrifugenbecher gebildet ist. Das zu verdichtende Medium ist der Blutbeutel mit der darin enthaltenen Blutflüssigkeit. Während des Zentrifugiervorganges wirkt das Halterungsteil als Kolben, das aufgrund der Zentrifugalkraft in axialer Richtung gegen den Blutbeutel gedrückt wird und dadurch in axialer Richtung im Zentrifugenbecher in Richtung auf den Boden des Zentrifugenbechers bewegt wird und gleichzeitig den Blutbeutel komprimiert. Nach vollendeter Trennung der Blutbestandteile im Blutbeutel während des Zentrifugiervorganges kann nun noch während des Laufs der Zentrifuge bzw. in der Auslaufphase ein Ventil im Anschlussschlauch des Blutbeutels fernbetätigt geöffnet werden, wodurch aufgrund der auf dem Blutbeutel herrschenden Kompressionskraft von Seiten des Halterungsteiles der nächst dem Anschlussteil des Blutbeutels befindliche Blutbestandteil durch die gegebene Durckeinwirkung über den Anschlusteil und den Anschlussschläuche in einem benachbarten Satelliten-Blutbeutel ausgelassen werden kann.

Das vorgeschlagene Halterungsteil erfüllt also eine Doppelfunktion, weil es zum einen das Anschlussteil des Blutbeutels vor dem Umknicken schützt und hierdurch Sedimentationsfallen vermeidet, und zum anderen gleichzeitig zur Gewinnung eines ausserordentlich reinen und qualitätsreichen Blutplasmas beiträgt, weil das Blutplasma noch während des Zentrifugiervorganges aus dem Blutbeutel abgelassen werden kann, wodurch die sich am Boden des Blutbeutels konzentrierenden Erythrozyten – aufgrund der noch vorherrschenden Zentrifugalkraft – zurückgehalten werden.

In einer bevorzugten Ausführungsform der vorliegenden Erfindung ist das Halterungsteil auf den Anschlussteil des Blutbeutels aufschiebbar. Es kann aber auch vorgesehen sein, dass das Halterungsteil auf den Anschlussteil des Blutbeutels aufklemmbar oder sonstwie lösbar befestigt ist. Sofern das Halterungsteil aufschiebbar auf den Anschlussteil des Blutbeutels ausgebildet ist, wird es hierbei bevorzugt, wenn das Halterungsteil aus einer horizontalen Platte besteht, in der eine schlitzförmige Ausnehmung angeordnet ist, wobei an den Begrenzungen der Ausnehmung ein in der Draufsicht U-förmig umlaufender, vertikaler Kragen angeformt ist, der an einer Seite der Platte und des Kragens einen Schlitz für den Durchtritt des Anschlussteils des Blutbeutels freilässt. Das Anschlussteil wird also bei aus dem Zentrifugenbecher herausgenommenem Blutbeutel auf den Anschlussteil des Blutbeutels seitlich aufgeschoben, wobei der Anschlussteil durch den Schlitz der Platte und des Kragens hindurchtritt, und dann etwa die schlitzförmige Ausnehmung ausfüllt.

Der so fertig bestückte Blutbeutel kann dann in den Zentrifugenbecher eingesetzt werden, wobei die Platte in ihrem Aussenumfang etwa dem Innenumfang des Zentrifugenbechers angepasst ist, so dass das Halterungsteil leicht und mit Spiel vertikal im Zentrifugenbecher bewegbar ist. Die horizontale Platte dient hierbei als «Kolben» in dem «Zylinder» des Zentrifugenbechers, während der an den Begrenzungen der Ausnehmung vertikal aufragende Kragen als Stützteil für den Schlussteil des Blutbeutels dient. Das Halterungsteil kann aber auch noch mit Hilfe von Schrauben oder anderen lösbaren Befestigungsmitteln mit dem Blutbeutel verbunden werden. Beispielsweise können im Kragen entsprechende Ausnehmungen vorgesehen sein, die mit zugeordneten Ausnehmungen im Blutbeutel fluchten. Durch die fluchtenden Ausnehmungen kann dann eine Schraube oder ein anderes lösbares Befestigungsteil hindurchgesteckt und am Halterungsteil befestigt werden. Wesentlich ist ferner, dass der Kragen des Halterungsteils auch noch als Aufwickelhaspel für die Anschlussschläuche des Blutbeutels dient. Damit die Anschlussschläuche während des Zentrifugiervorganges der Zentrifuge nicht beschädigt werden, werden die Anschlussschläuche um den Kragen des Halterungsteils herumgewickelt und am Kragen festgelegt. Die Festlegung dieser Anschlussschläuche erfolgt beispielsweise dadurch, dass in dem Kragen Einschnitte zur klemmenden Halterung der Anschlussschläuche angeordnet sind. Es muss nur sichergestellt sein, dass die Anschlussschläuche nicht abgeknickt werden, so dass während der Drehbewegung der Zentrifuge nach erfolgter Trennung der Blutbestandteile ein Blutbestandteil aus dem Blutbeutel über den Anschlussteil und die daran ansetzenden Anschlussschläuche abgelassen werden kann.

Wesentlich ist ferner, dass die Kraft, mit der der Blutbeutel von Seiten des Halterungsteils während des Zentrifugiervorganges komprimiert wird, zum einen von der Umdrehungszahl der Zentrifuge abhängt, zum anderen aber auch vom Gewicht des Halterungsteiles. Es ist hierbei vorgesehen, dass an dem Halterungsteil zusätzliche Gewichte lösbar befestigt sind, um je nach den Erfordernissen eine grössere oder kleinere Kompressionskraft auf den Blutbeutel bei gleichbleibender Rotationsgeschwindigkeit zu erhalten.

Im folgenden wird die Erfindung anhand von lediglich mehrere Ausführungswege darstellenden Zeichnungen näher erläutert.

Es zeigen:

Fig. 1 Schnitt durch den Zentrifugenbecher einer Zentrifuge gemäss der Linie I–I in Fig. 2 in einem ersten Ausführungsbeispiel

Fig. 2 Schnitt gemäss der Linie II–II in Fig. 1;

Fig. 3 Schnitt durch den Rotor einer Zentrifuge in einem zweiten Ausführungsbeispiel;

Fig. 4 Draufsicht auf den Rotor nach Fig. 3 mit teilweise weggebrochenem Deckel;

Fig. 5 Schnitt durch den Rotor einer Zentrifuge in einem dritten Ausführungsbeispiel;

Fig. 6 Draufsicht auf den Rotor nach Fig. 6 bei entferntem Deckel;

Fig. 7 eine weitere Ausführungsform bei der das Druckkissen werkstoffeinstückig mit dem Blutbeutel verbunden ist;

Fig. 8 schematisierter Mittenlängsschnitt durch die Hälfte eines Zentrifugenbechers mit Blutbeutel und darauf aufgesetztem Halterungsteil;

Fig. 9 Seitenansicht mit teilweisem Schnitt durch das Halterungsteil;

Fig. 10 Draufsicht auf das Halterungsteil gemäss Fig. 9;

Fig. 11 eine ähnliche Fig. 9 und Fig. 10 Ausbildung des Halterungsteiles in einer weiteren Ausführungsform;

Fig. 12 die Seitenansicht eines Halterungsteils in einer zweiten Ausführungsform;

Fig. 13 Draufsicht auf das Halterungsteil nach Fig. 12;

Fig. 14 perspektivische Darstellung eines Nut-Gehänges und eines Einsatzbechers zur Aufnahme von zwei Blutbeuteln und zwei Halterungsteilen.

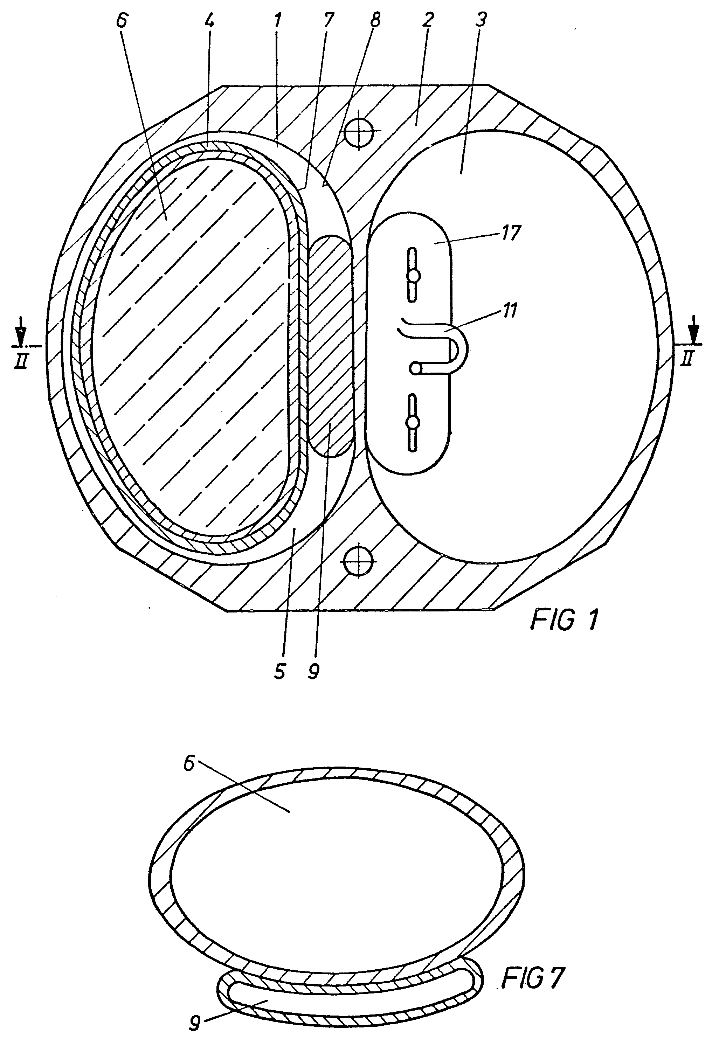

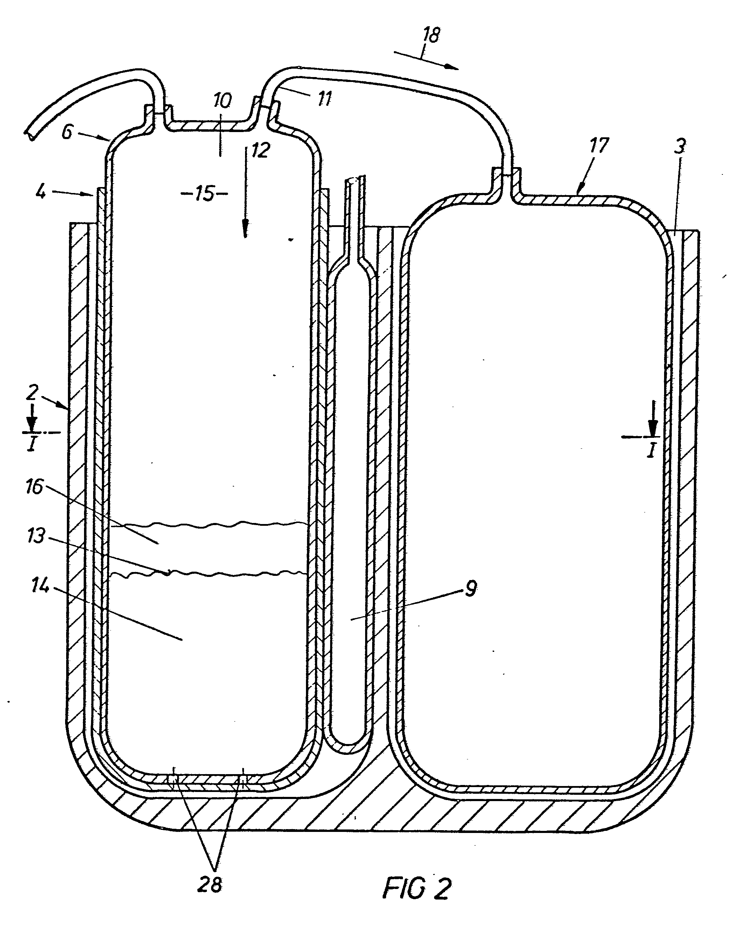

Im ersten Ausführungsbeispiel nach den Fig. 1 und 2 ist der Zentrifugenbecher 2 einer Zentrifuge mit zwei Kammern 1, 3 ausgestattet. In der ersten Kammer 1 ist der mit Blutflüssigkeit gefüllte erste Blutbeutel 6 eingesetzt. Der Blutbeutel 6 überragt hierbei mit seinem Kopfstück 10 die Oberkante des Zentrifugenbechers 2. Der Blutbeutel 6 ist in einem elastisch verformbaren Einsatzbecher 4 eingesetzt, der eine derartige Formgebung gemäss Fig. 1 hat, dass er mit seiner Aussenseite etwa an den Innenseiten der Kammer 1 anliegt und einen Zwischenraum 5 in Richtung zur Kammertrennwand freilässt, wo ein Druckkissen 9 eingesetzt ist. Das Druckkissen 9 liegt also einerseits an der Aussenseite 7 des Einsatzbechers 4 und anderseits an der Innenseite 8 des Zentrifugenbechers 2 an. Der Einsatzbecher 4 besteht aus einem Kunststoffmaterial, z.B. Polypropylen, oder aus einem elastisch biegbaren Metallbecher od.dgl. Um das Einsetzen des Blutbeutels 6 in den Einsatzbecher 4 zu gestatten, ist der Boden des Einsatzbechers mit Bohrungen 28 versehen, um die beim Einschieben des Blutbeutels 6 in den Einsatzbecher 4 verdrängte Luft ausströmen zu lassen.

Am Kopfstück 10 des Blutbeutels 6 ist ein Anschlussschlauch 11 angesetzt, der mit dem in der benachbarten Kammer 3 untergebrachtem zweiten (Satelliten-) Blutbeutel verbunden ist.

Vor Einleitung des Zentrifugiervorganges wird das Druckkissen 9 über seinen Anschlussschlauch mit Druck beaufschlagt. Hiebei wird es bevorzugt, wenn das Druckkissen 9 selbst mit einer Flüssigkeit, z.B. einer Salzlösung oder einer Wasser-Alkohol-Mischung, gefüllt ist. Das Druckkissen kann demzufolge auch in Kühl-Zentrifugen eingesetzt werden. Zur Druckbeaufschlagung der Druckflüssigkeit des Druckkissens 9 wird die Verwendung von Druckluft bevorzugt. Druckluft ist über entsprechend an der Zentrifuge angeordnete Rotationsverteiler wesentlich einfacher zuzuführen als entsprechende Druckflüssigkeit. Ferner spielen Undichtigkeiten bei Druckluftpumpen eine wesentlich geringere Rolle als bei Druckflüssigkeitspumpen.

Vor Beginn des Zentrifugiervorganges wird das Druckkissen 9 schon so weit komprimiert, dass der Blutbeutel 6 stramm und aufrecht stehend im Einsatzbecher 4 fixiert ist, so dass das Kopfstück 10 während des Zentrifugiervorganges mit Sicherheit nicht in Pfeilrichtung 12 umkippen kann.

Es wird dann der Zentrifugiervorgang eingeleitet. Nach einigen Minuten haben sich aufgrund der Zentrifugalkraft im Blutbeutel die verschiedenen Blutkomponenten getrennt. Im unteren Teil des Blutbeutels 6 sammeln sich die Erythrozyten (Erys) 14 an, die durch eine Trennlinie 13 von dem darüber lagernden Buffycoat 16 getrennt sind. Das Buffycoat selbst ist über eine weitere Trennlinie von dem darüber lagernden Blutplasma 15 getrennt.

Während des Zentrifugiervorganges – vorzugsweise aber während des Auslaufvorganges der Zentrifuge – wird das Druckkissen 9 weiter mit Druck beaufschlagt, so dass eine starke Volumenzunahme die Folge ist. Hierdurch wird der Einsatzbecher 4 elastisch zusammengedrückt, wodurch ebenfalls in gleichem Masse der Blutbeutel 6 komprimiert wird. Durch die entstehende Kompression wird zunächst das Blutplasma 15 in den Anschlussschlauch 11 hineingedrückt und läuft dort in Pfeilrichtung 18 in den daneben in der Kammer 3 angeordneten Blutbeutel 17. Auf diese Weise ist jetzt eine sehr genaue Trennung zwischen Blutplasma 15 und Buffycoat 16 möglich, weil die Trennlinien zwischen den einzelnen Blutkomponenten aufgrund der noch wirkenden Zentrifugalkraft sehr scharf ausgebildet bleiben und eine Verunreinigung des Blutplasmas durch die Erys auf jeden Fall vermieden wird.

In einer zeichnerisch nicht näher dargestellten Weiterbildung des Ausführungsbeispieles nach Fig. 1 und 2 ist vorgesehen, dass nicht nur ein zweiter Blutbeutel 17 in der Kammer 3 vorhanden ist, sondern noch ein zweiter oder dritter Blutbeutel 17. Diese Blutbeutel 17 wären alle durch den Anschlussschlauch 11 verbunden, wobei in dem jeweiligen Anschlussschlauch ein Zweiwegeventil vorhanden wäre. Hier ist es dann vorgesehen, dass zunächst durch Beaufschlagung des Druckkissens 9 und Komprimierung des Blutbeutels 6 das Blutplasma 15 in den ersten Blutbeutel 17 eingeleitet wird. Danach wird das Zweiwegeventil umgestellt, und das sich an das Blutplasma 15 anschliessende Buffycoat 16 wird in den zweiten Blutbeutel 17 eingeleitet. Es bleibt dann in dem Blutbeutel 6 nur noch die abzentrifugierten Erys enthalten.

Durch die Verwendung eines Einsatzbechers 4 ist der Blutbeutel 6 ausserordentlich gut handhabbar. Man kann nach dem eingangs beschriebenen, bekannten Verfahren auch so vorgehen, dass man nach dem Abzentrifugieren den Blutbeutel 6 zusammen mit dem Einsatzbecher entnimmt und den Einsatzbecher 4 selbst ausquetscht. Der Einsatzbecher wird dann zusammen mit dem darin eingesetzten Blutbeutel 6 in eine herkömmliche Quetschvorrichtung eingelegt. Hierbei wird es bevorzugt, wenn der Einsatzbecher 4 einen abgeflachten Bodenbereich hat, so dass dieser unmittelbar auf den Boden abgestellt werden kann. Der Einsatzbecher kann dann mit der Hand an seinem elastischen, oberen Bereich zusammengedrückt werden, wodurch jetzt ebenfalls auf besonders einfache Weise ohne Zuhilfenahme einer mechanischen Vorrichtung eine Trennung von Erys und Blutplasma erfolgen kann.

Aufgrund der Tatsache, dass der Einsatzbecher 4 oben offen ist und unten einen Bodenbereich hat, kann der Einsatzbecher praktisch nur im oberen Bereich zusammengedrückt werden. Hierdurch ergibt sich der Vorteil, dass die Erys am unteren Bodenbereich nicht zusammengequetscht werden und dadurch nicht in das Buffycoat 16 und in das Blutplasma 15 eindringen, so dass auch hiermit eine relativ reine Abtrennung der abzentrifugierten Blutkomponenten erreicht wird.

In einer Weiterbildung dieser Ausführung ist es vorgesehen, dass die Elastizität des Einsatzbechers 4 einstellbar ist. Hierzu sind entsprechende Vorrippungen oder Ausnehmungen vorgesehen, um je nach Art des gewünschten Anwendungsfalles eine mehr oder weniger grosse Zusammendrückbarkeit des Einsatzbechers 4 entweder nur im oberen Bereich oder im oberen Bereich bis zur Hälfte des Einsatzbechers usw. zu bekommen.

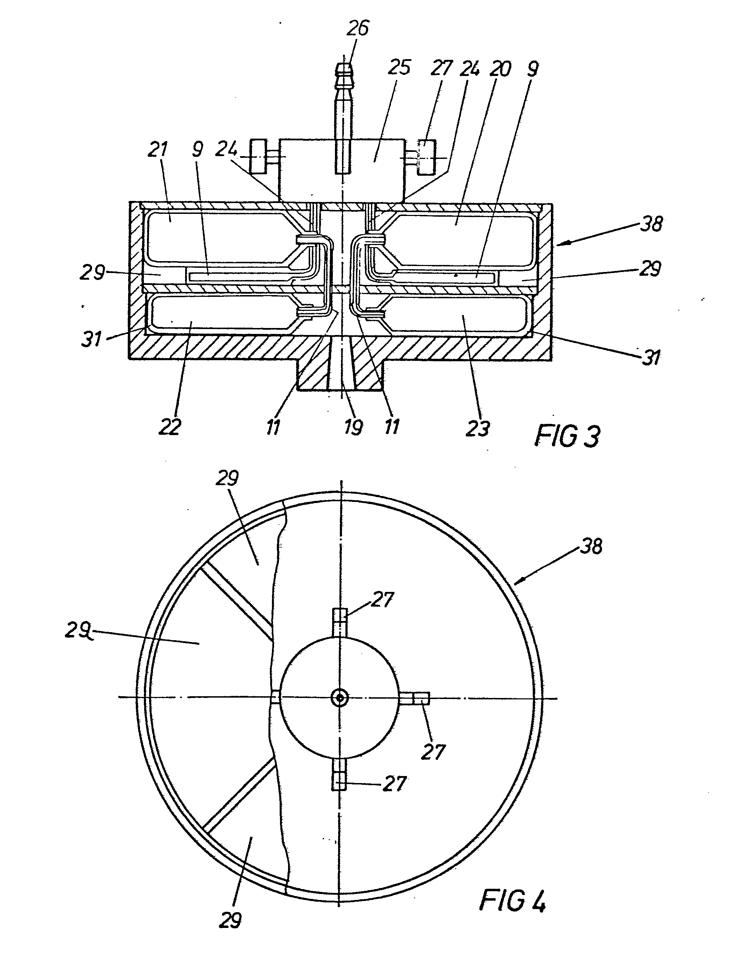

In Fig. 3 und 4 ist ein zweites Ausführungsbeispiel der vorliegenden Erfindung dargestellt, wo in einem Rotor 38 einer Zentrifuge horizontal übereinander liegende Kammern 29, 31 vorgesehen sind. Die Kammern sind entsprechend der Fig. 4 sektorförmig aufgeteilt, wobei in jeder Kammer 29 bzw. 31 ein entsprechend geformter Blutbeutel 20, 21 (mit Blutflüssigkeit gefüllt) bzw. ein (Satelliten-) Blutbeutel 22, 23 angeordnet sind.

Um eine Unwucht des Rotors 38 zu vermeiden, müssen die einander zugeordneten Blutbeutel 21, 22 bzw. 20, 23 symmetrisch in bezug zur Drehachse 19 angeordnet sein.

Entsprechende der oben stehenden Beschreibung sind die mit Blut gefüllten Blutbeutel 20, 21 über Anschlussschläuche 11 mit den (Satelliten-) Blutbeuteln 22, 23 verbunden.

Unterhalb der mit Blutflüssigkeit gefüllten Blutbeutel 20, 21 ist jeweils ein Druckkissen 9 angeordnet, das über Verbindungsschläuche 24 mit einem in der Drehachse 19 angeordneten Druckluft-Rotationsverteiler verbunden sind. Mit Position 27 sind die verschiedenen Anschlüsse und Ventile gekennzeichnet. Die Druckluft zur Druckbeaufschlagung der Druckkissen 9 wird über den Anschluss 26 dem Rotationsverteiler 25 zugeführt.

Nach Fig. 3 sind die Anschlussschläuche 11 so angeordnet, dass zunächst das Blutplasma 15 als leichtester Bestandteil der Blutflüssigkeit bei der Expansion des Druckkissens 9 aus dem Blutbeutel 20, 21 verdrängt wird. Die Anschlussschläuche 11 können aber auch im Bodenbereich der Blutbeutel 20, 21 angeordnet sein (also am radial auswärts liegenden Teil der Blutbeutel 20, 21), so dass zunächst bei Expansion des Druckkissens 9 die Erythrozyten 14 in die (Satelliten-) Blutbeutel 22, 23 eingeleitet wird.

Ebenso ist es möglich, mit entsprechenden Umschaltventilen die verschiedenen abzentrifugierten Komponenten auf verschiedene leere Blutbeutel 22, 23 zu verteilen.

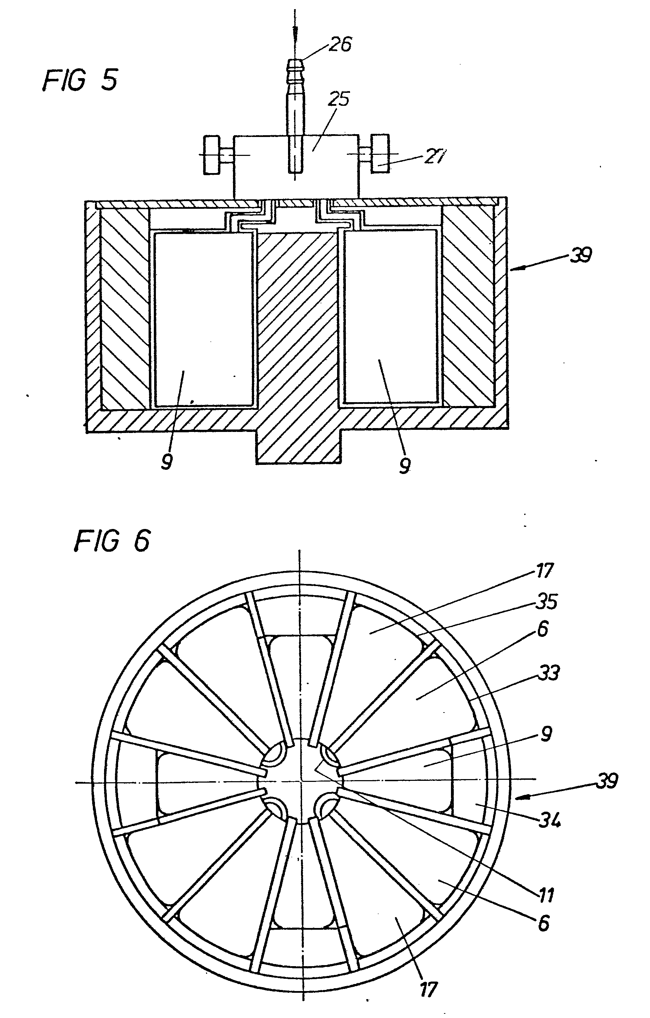

Das Ausführungsbeispiel nach den Fig. 5 und 6 unterscheidet sich von dem Ausführungsbeispiel der Fig. 3 und 4 dadurch, dass die Blutbeutel 6 mit den (Satelliten-) Blutbeuteln 17 auf einer Ebene eines Rotors 39 angeordnet sind. Die genannten Blutbeutel 6, 17 sind aufrecht stehend gemäss Fig. 5 angeordnet und wiederum über Anschlussschläuche mit einem Rotationsverteiler 25 verbunden. Die Blutbeutel 6, 17 sitzen hierbei in getrennten Kammern 33, 35 des Rotors 39 der Zentrifuge. Jeweils zwischen den mit Blutflüssigkeit gefüllten Blutbeuteln 6 ist ein Druckkissen 9 angeordnet, das sich an einer Abstützung 34 radial auswärts abstützt. Bei Expansion des Druckkissens 9 werden somit gleichzeitig zwei Blutbeutel 6 komprimiert, was hinsichtlich der geringeren Anzahl von Anschlussschläuchen und Anschlüssen am Rotationsverteiler 25 Vorteile bringt. Die Verbindung zwischen den Blutbeuteln 6 und 17 kann entsprechend der Fig. 6 sowohl an radial einwärts liegenden Ende der Blutbeutel als auch am radial auswärts liegenden Bodenbereich der Blutbeutel 6, 17 erfolgen.

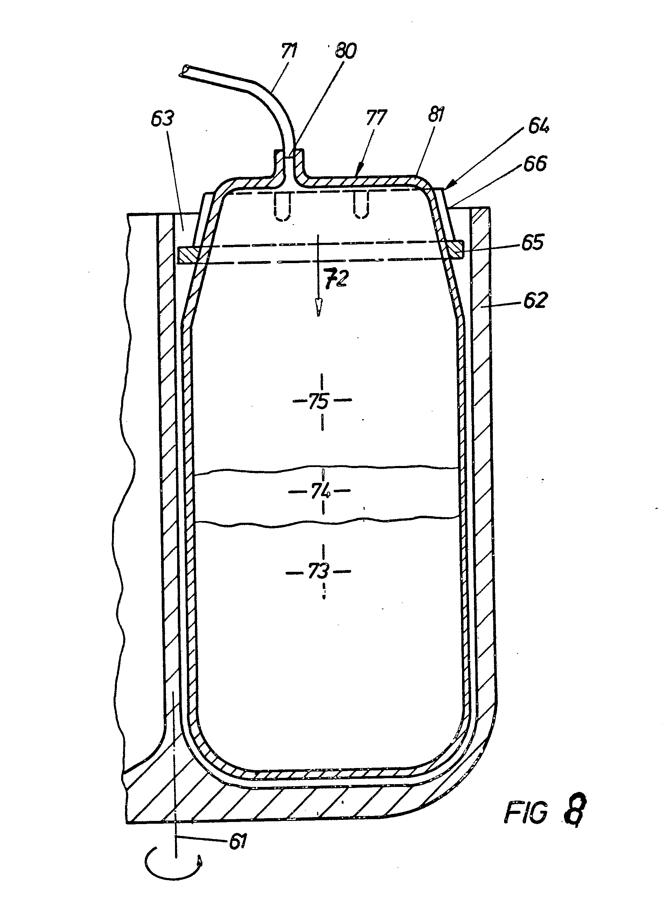

Fig. 7 zeit in Vergleich zur Fig. 1 und 2 eine andere Ausführungsform eines Blutbeutels 6. Hier ist gezeigt, dass der Blutbeutel 6 nicht nur zweistückig mit dem Druckkissen 9 ausgebildet sein kann (vgl. Fig. 1 und 2), sondern auch einstückig oder sogar werkstoffeinstückig. Im gezeigten Anwendungsfall kann das Druckkissen 9 an den Blutbeutel 6 angeschweisst oder angeklebt sein oder es kann auch die gezeichnete doppelte Wandung zwischen dem Druckkissen 9 und dem Blutbeutel 6 entfallen und durch eine einfache Wandung ersetzt sein. Ein solcher Blutbeutel 6 kann dann als Verkaufseinheit zusammen mit dem daran befestigten Druckkissen verkauft werden.

In Fig. 8 ist die rechte Hälfte eines Zentrifugenbechers dargestellt, der symmetrisch zur Drehachse 61 genau spiegelbildlich ausgebildet ist. Der Satelliten-Blutbeutel ist hierbei dann beispielsweise in dem nicht dargestellten linken Teil des Zentrifugenbechers angeordnet. In den zentralen Ausnehmungen 63 des rechts gezeigten Zentrifugenbechers 62 ist ein Blutbeutel 77 eingesetzt. Der Blutbeutel besteht in bekannter Weise aus einem Kunststoffmaterial, das aus zwei Folienblättern besteht, die an den umlaufenden Rändern zusammengeschweisst sind. An der oberen Seite des Blutbeutels ist der Anschlussteil 81 angeordnet, wo über ein oder mehrere Anschlussstücke 80 ein oder mehrere Anschlussschläuche 71 in den Blutbeutel 77 münden. Zusätzlich können am Anschlussteil 81 nicht näher dargestellte verschliessbare Einzelöffnungen oder sonstige Modifikationen vorhanden sein.

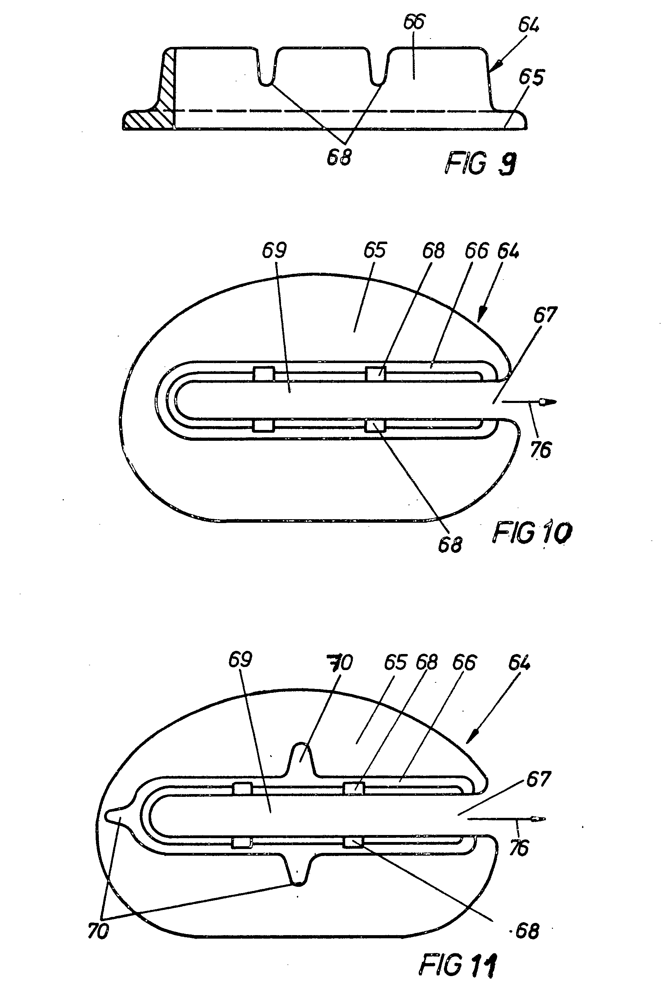

Auf das Anschlussteil 81 des Blutbeutels ist gemäss Fig. 8 das Halterungsteil 64 aufgeschoben, das aus einer umlaufenden, im wesentlichen horizontalen Platte 65 besteht, in der gemäss Fig. 9 und 11 iene schlitzförmige Ausnehmung 69 angeordnet ist, wobei an den Begrenzungen der Ausnehmung 69 ein in der Draufsicht U-förmig umlaufender, vertikaler Kragen 66 angeformt ist, der an einer Seite der Platte 65 und des Kragens 66 in den Schlitz 67 für den Durchtritt des Anschlussteils 81 des Blutbeutels 77 frei lässt.

Während des Zentrifugiervorganges erfolgt eine starke Zentrifugalkraft in Pfeilrichtung 72 auf den Blutbeutel und das Halterungsteil 64. Beide Teile werden somit gegen den Boden des Zentrifugenbechers 62 gepresst, wobei der Anschlussteil 81 des Blutbeutels 77 vor dem Umknicken in Pfeilrichtung 72 bewahrt wird, weil er beiderseits von dem umlaufenden, vertikalen Kragen 66 des Halterungsteiles 64 geführt wird. Das Halterungsteil 64 und das Anschlussteil 81 des Blutbeutels führen so während des Zentrifugiervorganges eine Axialbewegung in Richtung auf den Boden des Zentrifugenbechers 62 aus, wodurch der Blutbeutel 77 komprimiert wird.

Nach einer gewissen Laufzeit der Zentrifuge liegen dann die durch Abzentrifugieren zu gewinnenden Blutbestandteile getrennter Form im Blutbeutel 77 vor. In der Nähe des Bodens des Blutbeutels 77 befindet sich hierbei eine Schicht aus Erythrozyten, die über eine Grenzschicht (Buffycoat 74) in das Blutplasma 75 übergehen.

Während des Zentrifugiervorganges kann nun das Blutplasma 75 über den Anschlussteil 81 und den Anschlussschlauch 71 abgeleitet werden, weil das Halterungsteil 64 in Pfeilrichtung 72 aufgrund der noch herrschenden Zentrifugalkraft den Blutbeutel 77 komprimiert, und die hierdurch entstehende Druckerhöhung das Blutplasma 75 über den Anschlussschlauch 71 auslässt, wenn während des Zentrifugiervorganges das Ventil im Anschlussschlauch 71 geöffnet wird. Es wird hierdurch ein Blutplasma 75 ausserordentlich hoher Reinheit gewonnen, weil aufgrund der noch herrschenden Zentrifugalkraft die Erythrozyten 73 und das Buffycoat 74 am Boden des Blutbeutels 77 gehalten werden und eine unbeabsichtige Vermischung von Buffycoat 74 und von Erythrozyten 73 mit dem Blutplasma 75 auf diese Weise vermieden wird.

Die Fig. 9 und 10 zeigen, dass der Kragen 66 gleichzeitig als Aufwickelhaspel für den oder die Anschlussschläuche 71 dienen kann. Der Anschlussschlauch 71 wird hierbei um den Kragen 66 mehrfach herumgeschlungen und liegt dann parallel zur Fläche der Platte 65. Damit der Anschlussschlauch 71 nicht unbeabsichtigt sich von dem Kragen 66 löst, sind seitliche Einschnitte 68 in dem Kragen vorgesehen, in denen der Anschlussschlauch 71 klemmend festgelegt werden kann. Das gesamte Halterungsteil 64 wird in Pfeilrichtung 76 mit dem Schlitz 67 und die sich daran anschliessende Ausnehmung 69 auf den Anschlussteil 81 des Blutbeutels 77 seitlich aufgeschoben, bis das Halterungsteil 64 die in Fig. 8 dargestellte Lage erreicht.

Zur verbesserten Aufwicklung der Anschlussschläuche 71 auf dem Kragen 66 kann es gemäss Fig. 11 vorgesehen sein, dass an der oberen Begrenzung des Kragens 66 seitliche Ansätze 70 vorgesehen sind, die in ihrer Ebene parallel zur Platte 65 verlaufen und einen Abstand zur Platte 65 aufweisen. Es ergibt sich hiermit ein Aufwickelraum für den oder die Anschlussschläuche 71.

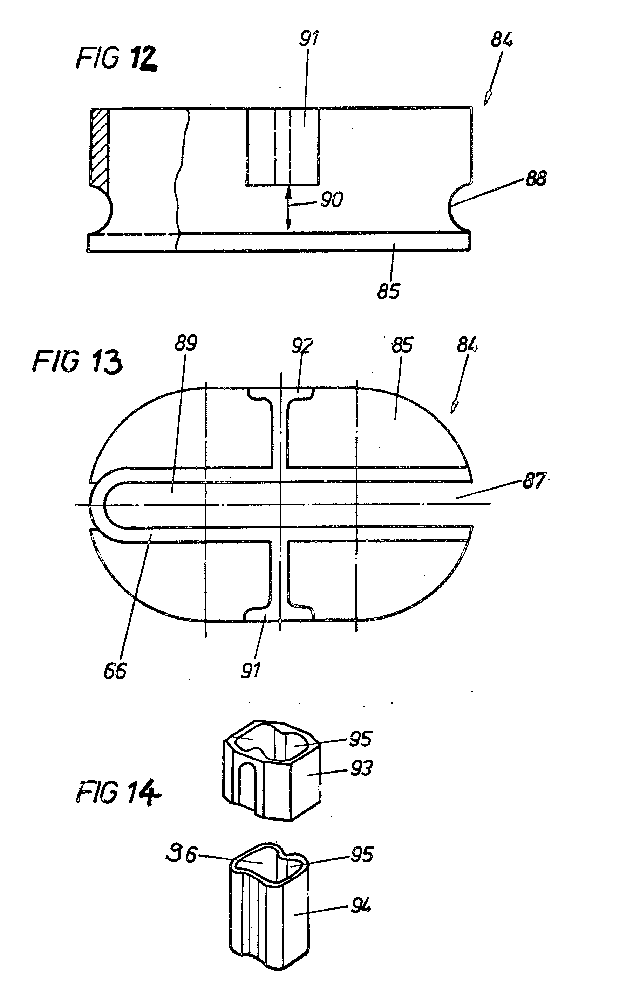

Die Fig. 12 und 13 zeigen die Ausführungsform eines zweiten Halterungsteils 84, das sich in wesentlichen Einzelheiten von dem vorher dargestellten Halterungsteil 64 unterscheidet. Das Halterungsteil 84 besteht ebenfalls aus einer horizontalen, oval geformten Platte 85, mit einem vertikal darauf aufragenden Kragen 66. Seitlich am oberen Rand des Kragens 66 sind im vorliegenden Ausführunsbeispiel in der Draufsicht T-förmige Ansätze 91, 92 angeformt, wobei der Schenkel des T-Profils am Kragen 66 des Halterungsteils 84 ansetzt. Hierdurch weisen die verbreiterten Flächen der Ansätze 91, 92 nach aussen und fluchten etwa mit den äusseren Begrenzungen der Platte 85. Hierdurch stützt sich das Halterungsteil 84 mit den Flächen der Ansätze 91, 92 sowohl an den Innenseiten des in Fig. 16 gezeigten Einsatzbechers 94 ab, als auch gegeneinander, an parallel gegenüberliegende, weitere Halterungsteil 84.

Gemäss Fig. 14 weist der Einsatzbecher 94 zwei Aufnahmeöffnungen 95, 96 auf, die jeweils Blutbeutel aufnehmen. In jede Aufnahmeöffnung 95, 96 wird ein Halterungsteil nach den Fig. 13 und 14 eingeschoben. Der Übergang von der einen Aufnahmeöffnung 95 zu anderen Aufnahmeöffnung 96 ist geöffnet.

Hier liegen die einander zugewandten Flächen der Ansätze 91 oder 92 der nebeneinander liegenden Halterungsteile 84 an, und stützen sich gegenseitig ab.

Der Einsatzbecher 94 wird dann in das Nutgehänge 93 eingehängt und das Nutgehänge 93 wird in den Schleuderkopf der Zentrifuge eingesetzt.

Wesentlich bei diesem Ausführungsbeispiel ist also, dass mehrere Halterungsteile 84 in einem Einsatzbecher 94 verwendet werden, und die Halterungsteile 84 sich gegenseitig abstützen.

Wesentlich ist ferner, dass gemäss den Fig. 12 und 13 zwischen der Oberseite der Platte 85 und der Unterkante der Ansätze 91, 92 ein Aufnahmeraum 90 für die Aufnahme der Anschlussschläuche der Blutbeutel ausgebildet ist. Die Anschlussschläuche werden somit in die radial umlaufende Nut 88 des Halterungsteils 84 aufgewickelt.

Zur Befestigung des Blutbeutels vom Halterungsteil wird gemäss der oben stehenden Beschreibung das Halterungsteil 84 mit dem Schlitz 87 auf den Blutbeutel aufgeschoben. Der obere Teil des Blutbeutels greift dann hierdurch durch die Ausnehmung 89 des Kragens 66 hindurch.

**Patentansprüche**

1. Zentrifuge mit wenigstens zwei Kammern (1, 3) für miteinander über einen Schlauch verbundene Blutbeutel, wobei in einer der Kammern (1) zusätzlich ein mit Druck beaufschlagbarer Verdrängungskörper eingesetzt ist, der bei Beaufschlagung mit Druck das Volumen des ihm zugeordneten Blutbeutels (6) verringert und dabei dessen Inhalt über den Schlauch (11) in den oder die weiteren Blutbeutel drückt, dadurch gekennzeichnet, dass der Verdrängungskörper als Druckkissen (9) ausgebildet ist, das in einer der Kammern (1) neben dem Blutbeutel (6) angeordnet ist.

2. Zentrifuge nach Anspruch 1, dadurch gekennzeichnet, dass der Blutbeutel (6) in einen verformbaren Einsatzbecher (4) eingesetzt ist.

3. Zentrifuge nach Anspruch 1, dadurch gekennzeichnet, dass der mit Blutflüssigkeit gefüllte Blutbeutel (6) mit dem Druckkissen (9) werkstoffeinstückig ist oder durch Verkleben, Verschweissen und ähnliches mit diesem verbunden ist (Fig. 7).

4. Zentrifuge nach einem der Ansprüche 1 bis 3, dadurch gekennzeichnet, dass das Druckkissen (9) mit einer Flüssigkeit gefüllt ist und die Druckbeaufschlagung mittels Druckluft erfolgt.

5. Zentrifuge nach einem der Ansprüche 1 bis 4, dadurch gekennzeichnet, dass dem ersten mit Blutflüssigkeit gefüllten Blutbeutel (6; 20, 21) mehrere zweite (Satelliten-) Blutbeutel (17; 22, 23) zugeordnet sind und dass nach der Verdrängung der ersten abzentrifugierten Komponente in den einen (Satelliten-) Blutbeutel (17; 22, 23) die anderen Komponenten in die anderen (Satelliten-) Blutbeutel (17; 22, 23) verdrängbar sind.

6. Zentrifuge mit mehreren radial ausserhalb der Rotationsachse angeordneten Aufnahmen für Blutbeutel, die über eine Leitung mit einem fernbetätigten Ventil miteinander verbunden sind, nach einem der Ansprüche 1 bis 5, dadurch gekennzeichnet, dass die Blutbeutel (20, 23) auf demselben Radius der Zentrifuge angeordnet sind.

7. Zentrifuge mit einem in einen Zentrifugenbecher lose einsetzbaren Teil zur Halterung eines Blutbeutels im Zentrifugenbecher, die an dem oberen Anschlussteil des Blutbeutels angreift und diesen Anschlussteil vor dem Unknicken während des Zentrifugiervorganges schützt, nach einem der Ansprüche 1 bis 6, dadurch gekennzeichnet, dass das Teil aus einem Halterungsteil (64, 84) aus starrem Material mit einer Ausnehmung (69, 89) für den Durchtritt des Anschlussteils (81) besteht, der auf den Blutbeutel (77) aufgesetzt ist und dessen Aussenumfang dem Innenumfang des Zentrifugenbechers (62) entspricht.

8. Zentrifuge nach Anspruch 7, dadurch gekennzeichnet, dass am oberen, umlaufenden Rand des Kragens (66) seitliche, etwa zur Platte (65, 85) parallele Ansätze (70, 91, 92) angeformt sind (Fig. 11–13).

9. Zentrifuge nach einem der Ansprüche 7 und 8, dadurch gekennzeichnet, dass in den Kragen (66) Einschnitte (68) zur klemmenden Halterung der Anschlussschläuche (71) angeordnet sind.

10. Zentrifuge nach einem der Ansprüche 7 bis 9, dadurch gekennzeichnet, dass an der Halterung (64) zusätzliche Gewichte lösbar befestigt sind.

11. Zentrifuge nach einem der Ansprüche 7 bis 10, dadurch gekennzeichnet, dass am oberen Rand des Kragens (66) des Halterungsteils (84) seitliche Ansätze (91, 92) angeformt sind, deren freie Enden etwa mit der Begrenzung der Platte (85) fluchten (Fig. 12, 13).

12. Zentrifuge nach Anspruch 11, dadurch gekennzeichnet, dass die seitlichen Ansätze (91, 92) in der Draufsicht als T-Profil ausgebildet sind und der T-Schenkel am Kragen (66) ansetzt (Fig. 12, 13).

13. Zentrifuge nach Anspruch 11 und 12, dadurch gekennzeichnet, dass zwischen der Oberseite der Platte (85) und der Unterkante der Ansätze (91, 92) ein Aufnahmeraum (90, 88) zum Aufwickeln der Anschlussschläuche ausgebildet ist (Fig. 12).

**Claims**

1. Centrifuge having at least two chambers (1, 3) for blood bags interconnected by a connecting tube, in which one of the chambers (1) ist in addition provided with a pressure-subjectable displacement body, which under pressure reduces the volume of its associated blood bag (6) and squeezes out the content into that or further blood bags by way of the tube (11), characterized in that the displacement body is formed as a pressure pad (9), which is placed inside one of the chambers (1) adjacent the blood bag (6).

2. Centrifuge as set forth in claim 1, characterized in that the blood bag (6) is placed inside a deformable insert cup (4).

3. Centrifuge as set forth in claim 1, characterized in that the blood bag (6) filled with blood fluid and the pressure pad (9) are made in one workpiece or joined together by means of cementing, welding or similar (Fig. 7).

4. Centrifuge as set forth in one of claims 1 throug 3, characterized in that the pressure pad (9) is filled with a fluid and pressure is applied by means compressed air.

5. Centrifuge as set forth in one of claims 1 through 4, characterized in that the first with blood fluid filled blood bag (6, 20, 21) has associated to it several second (satellite) blood bags (17, 22, 23), and, that after the displacement of the first away centrifuged components into one of the (satellite) blood bags (17, 22, 23) the other components are displaceable into the other (satellite) blood bags (17, 22, 23).

6. Centrifuge having several radially outward the rotational axis arranged inserts for blood bags, which are by means of a tube interconnected with a remote-controllable valve as set forth in one of claims 1 through 5, characterized in that the blood bags (20, 23) are arranged in the same radius as the centrifuge.

7. Centrifuge having a loose into a centrifugal cup insertable part for retaining one of the blood bags inside the centrifugal cup, which engages the upper connecting part of the blood bag and prevents that connecting part from becoming kinked during the centrifugal process as set forth in one of claims 1 through 6, characterized in that the part consists of a retaining part (64, 84) made from rigid material having a recess (64, 84) for the interpenetration of the connecting part (81), which is set on atop the blood bag (77), and, which outer circumference is adapted to fit the inner circumference of the centrifugal cup (62).

8. Centrifuge as set forth in claim 7, characterized in that at the upper circumferential edge of the collar (66) lateral nearly parallel to the plate (65, 85) arranged lugs (70, 91, 92) are formed.

9. Centrifuge as set forth in claim 7 and 8, characterized in that the collar (66) is provided with cuts (68) for supporting the connection tubes (71) in a clamping manner.

10. Centrifuge as set forth in one of claims 7 through 9, characterized in that at the support (64) additional weights are detachable mounted.

11. Centrifuge as set forth in one of claims 7 through 10, characterized in that at the upper edge of the collar (66) of the supporting part (84) lateral lugs (91, 92) are formed on, whose free ends are nearly in alignment with the boundery of the plate (85) (Figs. 12, 13).

12. Centrifuge as set forth in claim 11, characterized in that the lateral lugs (91, 92) are in top view T-shaped formed, and, that the T-leg initiates at the collar (66) (Figs. 12, 13).

13. Centrifuge as set forth in claim 11 and 12, characterized in that between the top surface of the plate (85) and the bottom edge of the lugs (91, 92) a receiving space (90, 88) for winding up the connection tubes is formed (Fig. 12).

**Revendications**

1. Centrifugeuse qui comprend au moins deux chambres (1, 3) destinées à recevoir des poches à sang reliées l'une à l'autre par un tuyau, tandis que dans l'une des chambres (1) est, en outre, placé un corps de refoulement sur lequel une pression peut être exercée et qui, lors de l'application de cette pression, diminue le volume de la poche à sang (6) associée et, ce faisant, refoule le contenu de celle-ci, à travers le tuyau (11) dans la ou dans les autres poches à sang, caractérisée en ce que le corps de refoulement présente la forme d'un coussin de pression (9), disposé dans la chambre (1) à côté de la poche à sang (6).

2. Centrifugeuse selon la revendication 1, caractérisée en ce que la poche à sang (6) est logée dans un godet amovible déformable (4).

3. Centrifugeuse selon la revendication 1, caractérisée en ce que la poche à sang (6) remplie d'un liquide sanguin est venue de matière avec le coussin de pression (9) ou est reliée par collage, soudage ou autres moyens analogues, avec celui-ci (fig. 7).

4. Centrifugeuse selon l'une des revendications 1 à 3, caractérisée en ce que le coussin de pression (9) est rempli d'un liquide et l'application de la pression s'effectue au moyen d'air comprimé.

5. Centrifugeuse selon l'une des revendications 1 à 4, caractérisée en ce qu'à la première poche à sang (6; 20, 21) remplie d'un liquide sanguin, sont associées plusieurs deuxièmes poches à sang (satellites), (17; 22, 23) et en ce qu'après le refoulement du premier composant séparé par la centrifugation dans l'une des poches à sang (satellites), (17; 22, 23), les autres composants peuvent être refoulés dans les autres poches à sang (satellites), (17; 22, 23).

6. Centrifugeuse comportant plusieurs logements disposés radialement à l'extérieur de l'axe de rotation, pour recevoir des poches à sang qui sont reliées les unes aux autres par un conduit comportant une soupape télécommandée, selon l'une des revendications 1 à 5, caractérisée en ce que les poches à sang (20, 23) sont disposées sur un même rayon de la centrifugeuse.

7. Centrifugeuse comportant un élément pouvant être introduit librement dans un godet de la centrifugeuse pour le maintien d'une poche à sang dans ledit godet de centrifugeuse, élément qui s'applique à la partie de raccordement supérieure de la poche à sang et qui protège cette partie de raccordement du pliage pendant l'opération de centrifugation, selon l'une des revendications 1 à 6, caractérisée en ce que ledit élément se compose d'un élément de support (64, 84) en une matière rigide, qui présente un évidement (69, 89) pour le passage de la partie de raccordement (81) et qui s'enfile sur la poche à sang (77); le pourtour extérieur de cet élément de support correspondant au pourtour intérieur du godet (62) de la centrifugeuse.

8. Centrifugeuse selon la revendication 7, caractérisée en ce que, sur le bord périphérique supérieur du col (66), sont formés des prolongements latéraux (70, 91, 92), (figs 11–13) approximativement parallèles à la plaque (65, 85).

9. Centrifugeuse selon l'une des revendications 7 et 8, caractérisée en ce que dans les cols (66) sont formées des incisions (68) pour le maintien par pincement des tuyaux de raccordement (71).

10. Centrifugeuse selon l'une des revendications 7 à 9, caractérisée en ce que des poids supplémentaires sont fixés de façon amovible au support (64).

11. Centrifugeuse selon l'une des revendications 7 à 10, caractérisée en ce que sur le bord supérieur du col (66) de l'élément de support (84) sont formés des prolongements latéraux (91, 92), dont les extrémités libres sont approximativement alignées avec le bord délimitant la plaque (85), (figs 12–13).

12. Centrifugeuse selon la revendication 11, caractérisée en ce que les prolongements latéraux (91, 92) ont, vus en plan, un profil en T et le pied du T s'applique contre le col (66) (figs 12–13).

13. Centrifugeuse selon les revendications 11 et 12, caractérisée en ce qu'entre la face supérieure de la plaque (85) et le bord inférieur des prolongements (91, 92), est formé un espace de réception (90, 88) pour l'enroulement des tuyaux de raccordement (fig. 12).

FIG 1

FIG 7

FIG 2

FIG 3

FIG 4

FIG 5

26

25

21

39

9          9

FIG 6

17

35

6

33

9

39

34

11

6

17

17

FIG 8

FIG 9

FIG 10

FIG 11

FIG **12**

FIG **13**

FIG **14**